# EUROPÄISCHE PATENTSCHRIFT

(11) **EP 0 774 982 B1**
(45) Veröffentlichungstag und Bekanntmachung des Hinweises auf die Patenterteilung: **18.03.1998**
(21) Anmeldenummer: 95932673.7
(22) Anmeldetag: 06.09.1995
(51) Int. Cl.: A61L 27/00

(54) **VERFAHREN ZUR ELEKTROABSCHEIDUNG VON HYDROXYAPATITSCHICHTEN**
METHOD FOR THE ELECTRODEPOSITION OF HYDROXYAPATITE LAYERS
PROCEDE D'ELECTRODEPOSITION DE COUCHES D'HYDROXYAPATITE

(30) Priorität: 07.09.1994 DE 4431862
(43) Veröffentlichungstag der Anmeldung: 28.05.1997
(73) Patentinhaber: DOT DÜNNSCHICHT- UND OBERFLÄCHENTECHNOLOGIE GMBH, 18059 Rostock (DE)
(72) Erfinder: TELLER, Joachim, Dr., D-18276 Mistorf (DE); NEUMANN, Hans-Georg, Prof. Dr., D-18146 Rostock (DE)
(74) Vertreter: Schnick, Achim
(86) Internationale Anmeldenummer: EP9503499
(87) Internationale Veröffentlichungsnummer: WO9607438

(56) Entgegenhaltungen:
- WO-A-93/21969
- CA-A- 2 073 781

## Beschreibung

Die Erfindung betrifft ein Verfahren zur Elektroabscheidung von Hydroxylapatitschichten mit der allgemeinen Zusammensetzung Cax+9(PO4)x+5(HPO4)1-x(OH)x+1, wobei x für Werte zwischen Null und 1 steht, auf Metall- und Keramikflächen. Auf diese Art und Weise beschichtete Materialien finden ihren Einsatz in Form biokompatibler Implantate in der Human- und Veterinärmedizin, die sich besonders durch hohe Einwachsraten auszeichnen. Zur Herstellung von Hydroxylapatit- und anderen Calciumphosphat-Beschichtungen sind bereits verschiedene Verfahren beschrieben worden, die allerdings alle mit gewissen Mängeln behaftet sind.

Zu den bereits etablierten Verfahren gehöhrt das Plasma-Spraying von Hydroxylapatit (Radin, S.R. et al.: J. Mater. Sci. Mater in Medicine 1992, 33), bei dem es jedoch bei einem vergleichsweise hohen technischen Aufwand auf Grund des zeilen- bzw. seitenweisen Schichtauftrags nicht gelingt, gleichmäßige Beschichtungen in Bezug auf Schichtdicke, -haftung und -homogenität bei etwas komplizierteren Substratgeometrien zu erzeugen. Hydroxylapatit-Überzüge mit durchweg geringer Schichthaftung können durch Trocknung (Ducheyne, P. et al.: J. Biomed. Mater. Res. 1980, 225) , konventionelle Sinterung (Arita, I.H. et al.: Mater. Res. Symp. V (San Francisco, CA) 1993, Abstr. V2.2) oder Mikrowellensinterung (Agrawal, D.K. et al.: Mat. Res. Soc. Symp. Proc. Vol. 269, 231 (1992)) von naßchemisch erzeugtem bzw. aus Suspensionen abgeschiedenem Hydroxylapatit hergestellt werden. Aufwendige (Vakuum)sinterprozesse zur Erzeugung gleichmäßiger Beschichtungen sind auch für das Sol-Gel-Verfahren (Brendel, T. et al.: J. Mater. Sci. Mater. in Medicine 1992, 175) und ein Elektrophorese-Verfahren (Ducheyne, P. et al.: Biomaterials 1990, 244) charakteristisch. Ein hoher technischer Aufwand für den eigentlichen Beschichtungsvorgang kennzeichnet solche Verfahren, die auf der Sputter-Technik (Lacefield, W.R.: Ann. New York Acad. Sci. 1988, 72) bzw. dem Pulsed-Laser-Deposition-Prozeß (Cotell, C.M. et al.: J. Appl. Biomater. 1992, 87) beruhen. Technologisch leichter zu realisieren ist hingegen ein elektrochemisches Verfahren (WO 92/ 13984), daß für die Calciumphosphat-Beschichtung mechanisch vorbehandelter Oberflächen von Titan(legierungs)substraten geeignet ist. Gegenüber dem ursprünglichen Gleichstromverfahren (WO 92/ 13984) soll durch die Anwendung von gepulstem Gleichstrom (JP 5 285 212; Shirkhanzadeh, M.: J. Mater. Sci. Letters 1993, 16) (20 s Ein-Aus-Takt; 3,5 V) bzw. durch die Kombination von Gleichstrom- und On-Off-Betriebsweise (CA 2 073 781) ein gleichmäßigeres Wachstum der Beschichtung erzielt werden. Die Haftfestigkeit der Calciumphosphatschicht auf dem Titansubstrat wird dadurch allerdings nicht verbessert.

In der WO 93/21969 A1 ist ein Verfahren beschrieben, bei dem ein Metall- oder Keramiksubstrat im Sol-Gel-Verfahren mit einer titanhaltigen Beschichtung versehen wird. Die Sol-Gel-Beschichtung bei diesem Verfahren ist so konzipiert, daß nur durch *in vitro*-Lösungen, z.B. simulierte Körperflüssigkeit (SBF), oder durch *in vivo*-Bedingungen Apatitschichten aufwachsen, was erfahrungsgemäß nur über längere Zeiträume realisierbar ist und zu einer unregelmäßigen Beschichtung ohne wesentliche Konzentrationsgradienten führt.

Durch die US 4818512 ist schließlich auch ein Verfahren zur Beschichtung eines Metalls, wie beispielsweise Titan, einer Titanlegierung, Stahl oder einer Cobalt-Chrom-Legierung mit Calciumphosphat bekannt. Dabei wird vorzugsweise das Plasmaspritzen von Hydroxylapatit auf eine chemisch-passivierend vorbehandelte Oberfläche durchgeführt. Plasmagespritzte Hydroxylapatit-Schichten auf passivierten Oberfläxhen besitzen verfahrensbedingt andere physikalische, chemische und physiologische Eigenschaften als elektrochemisch abgeschiedene Hydroxylapatit-Schichten und sind zur Lösung der der Erfindung zugrunde liegenden Problematik ungeeignet.

Mit der vorliegenden Erfindung wird die Aufgabenstellung verfolgt, ein Verfahren zur Abscheidung von Hydroxylapatit auf Metall-, Legierungs- und Keramikoberflächen zu entwickeln, daß sich durch eine gleichmäßige, gut haftende Beschichtung mit optimaler Schichtdicke und geringen amorphen Anteilen bei vergleichsweise geringem gerätetechnischen, energetischen, zeitlichen und Materialaufwand auszeichnet.

Diese Aufgabe wird erfindungsgemäß dadurch gelöst, daß die eigentliche elektrochemische Beschichtung unter Verwendung eines calcium- und phosphathaltigen Elektrolyten mittels gepulstem Gleichstrom kombiniert wird mit einer Vorbeschichtung durch Sol-Gel-Prozesse unter Verwendung von Alkoxiden eines Elementes oder mehrer Elemente der 3.-5. Haupt- oder Nebengruppe des Periodensystems oder Mischungen solcher Alkoxide. Durch diese Kombination werden Konzentrationsgradienten zwischen einem oder mehreren chemischen Elementen des Grundmaterials und der Hydroxylapatit-Schicht erzeugt, wodurch eine wesentliche Erhöhung der Haftfestigkeit der Beschichtung erzielt wird. Die zu beschichtenden Substrate sollten in Hinblick auf die Anwendungsgebiete der Erfindung selbst über eine genügende Biokompatibilität verfügen. Nach der Erfindung werden als Metalle vorzugsweise Titan, Gold, Platin und Legierungen, insbesondere Edelstähle, CoCr28Mo und TiAl6V4 verwendet, als keramische Werkstoffe vorzugsweise Oxide, Nitride, Oxynitride bzw. Carbonitride, die in ihrer Zusammensetzung ein oder mehrere Metalle der 4. und 5. Nebengruppe des Periodensystems der chemischen Elemente enthalten. Die zu beschichtenden Substrate bestehen entweder ganz oder teilweise aus den vorstehend angegebenen Werkstoffen bzw. diese Werkstoffe sind in Form von ein- oder mehrschichtigen Überzügen auf Grundkörpern aus Metallen, Legierungen, Keramiken, Gläsern oder Kunststoffen Bestandteil der Substrate.

Bei der Sol-Gel-Beschichtung können zusätzlich chemische Verbindungen, die Calcium- und/oder Phosphationen enthalten, verwendet werden.

Die Sol-Gel-Beschichtung erfolgt durch Sprühen, Ultraschallvernebelung oder nach der Dip-Coating-Methode.

Es ist ebenso möglich, die Vorbehandlung durch eine Aufeinanderfolge von (elektro)chemischer Passivierung, wobei vorzugsweise verdünnte Phosphorsäure oder Mischungen, die diese enthalten, zum Einsatz gelangen und Sol-Gel-Beschichtung vorzunehmen.

Die elektrochemische Beschichtung der vorbehandelten Substrate erfolgt in einer Elektrolyse- zelle, die mit den üblichen Vorrichtungen zur Temperierung und Durchmischung des Elektrolyten, der Calcium- und (Hydrogen)phosphationen in einem molaren Verhältnis von annähernd 1,5 bis 1,7 enthält, einer inerten Anode, beispielsweise aus Kohlenstoff, Gold oder Platin, und dem Substrat selbst als Katode ausgestattet ist, unter Anwendung von gepulstem Gleichstrom mit einer Pulsfrequenz zwischen 1 und 300 Hz, vorzugsweise von 50 Hz bei gleichen Ein-Aus-Intervallen, und einer Spannung zwischen 2 und 10 V. Für die Güte der Beschichtung optimale Arbeitstemperaturen liegen im Bereich von 20-40°C. Üblicherweise werden die so präparierten Beschichtungen mit deionisiertem Wasser gewaschen und und im gegebenenfalls vorgewärmten Luftstrom getrocknet Weiterführend ist eine Temperung der beschichteten Substrate bei Temperaturen zwischen 300 und 500°C möglich aber nicht un- bedingt erforderlich. Wird eine derartige thermische Behandlung vorgenommen, so ist damit der nützliche Effekt verbunden, daß die Beschichtung anteilig Carbonate enthält.

Die erfindungsgemäß hergestellten Beschichtungen sind nach den üblichen Methoden sterilisierbar.

Die Schichtdicke der Hydroxylapatit-Abscheidung ist über die Elektrolytkonzentration und die übrigen Vorbehandlungs- und Prozeßparameter variierbar und liegt in typischen Anwendungsfällen in einer Größenordnung von 5 bis 25 µm. Damit wird eine Belegungsdichte der Beschichtung von 1 bis 3 mg/cm² erzielt.

Der Vorteil der Erfindung besteht darin, daß nach dem vorstehendem Verfahren Hydroxylapatit-Abscheidungen auf Metall- und Keramikoberflächen vorgenommen werden können, wie sie für einen Einsatz als biokompatible Implantate in der Human- und Veterinär- medizin erforderlich sind. Desweiteren wird durch eine passivierende und/oder Sol-Gel- Vorbehandlung der zu beschichtenden Substrate das Aufbringen gleichmäßiger, gut haftender Hydroxylapatit-Schichten mit geringem amorphen Anteil und optimaler Schichtdicke gewährleistet. Die Elektroabscheidung von Hydroxylapatit mittels gepulsten Gleichstrom repräsentiert zudem ein Verfahren, daß mit einem vergleichsweise geringen gerätetechnischen, energetischen, zeitlichen und Materialaufwand realisierbar ist.

Die Erfindung soll anhand der nachfolgenden Beispiele näher erläutert werden.

### Ausführungsbeispiele

### Beispiel 1

Eine Ti-Platte mit den Abmessungen 80x10x1,5 mm wird mit Isopropanol/Aceton (1 : 1) im Ultraschallbad gereinigt . Die Oberfläche wird einer zweimaligen Sol-Gel-Beschichtlung unter Verwendung einer Lösung bestehend aus 0,05 mol/l Ti(iso-C₃H₇O)₄ , 0,05 mol/l Si(OC₂H₅)₄ ünd 0,1 mol/l HCl in Form von 37%iger Salzsäure bei der ersten Behandlung und 0,05 mol/l Phosphorsäure in Form von 85%iger Phosphorsäure bei der zweiten Behandlung in iso-Propanol nach der Dip-coating-Technik unterzogen (Ziehgeschwindigkeit 1 cm/min). Die elektrochemische Abscheidung von Hydroxyapatit findet nach ca. 6 Stunden in einer Glaszelle mit 100 ml Fassungsvermögen unter Verwendung der vorbeschichteten Ti-Platte als Katode und einer Kohlenstoffanode statt. Als Elektrolyt wird eine Lösung bestehend aus 0,1 mol/l NH₄H₂PO₄ und 0,167 mol/l CaCl₂, die bei 22 °C magnetisch gerührt wird, verwendet. Innerhalb von 10 Minuten scheidet sich beim Anlegen von gepulstem 200 Hz-Gleichstrom (gleiche Ein- und Auszeiten) und einem Potential von 6 V eine Hydroxyapatitschicht auf der Ti-Platte ab. Die beschichtete Platte wird nachfolgend zweimal mit deionisiertem Wasser gewaschen und in einem warmen Luftstrom in ca. 5 Minuten getrocknet. Nach diesem Verfahren wird eine Beschichtung mit einer Belegungsdichte von 2,16 mg/cm² Elektrodenoberfläche erzeugt.

### Beispiel 2

Eine Ti-Platte wie in Beispiel 1 wird bei einer Stromdichte von 10 mA/cm² und einem Potential von ca. 12 V in 5%iger Phosphorsäure anodisch passiviert und einer Sol-Gel-Beschichtung unter Verwendung einer Lösung bestehend aus 0,05 mol/l Ti(iso-C₃H₇O)₄ , 0,04 mol/l Si(OC₂H₅)₄, 0,01 mol/l Phosphorsäuretriethylester und 0,1 mol/l HCl in Form von 37%iger Salzsäure in Isopropanol durch Besprühen unterzogen. Die Hydroxyapatitabscheidung erfolgt wie in Beispiel 1 unter Verwendung eines Elektrolyten bestehend aus 0,1 mol/l Ca(NO₃)₂ und 0,06 mol/l Na₂HPO₄.

### Beispiel 3

Eine Ti-Platte wie in Beispiel 1 wird einer zweimaligen Sol-Gel-Behandlung unter Verwendung einer Lösung bestehend aus 0,05 mol/l Ti(iso-C₃H₇O)₄ , 0,05 mol/l Si(OC₂H₅)₄ ünd 0,1 mol/l HCl in Form von 37%iger Salzsäure in iso-Propanol nach der Dip-coating-Technik unterzogen (Ziehgeschwindigkeit 1 cm/min). Die elektochemische Abscheidung von Hydroxyapatit erfolgt nach ca. 6 Stunden unter den Bedingungen des Beispiels 1, jedoch bei 40 °C.

### Beispiel 4

Eine Ti-Platte wird wie in Beispiel 3 behandelt. Zusätzlich erfolgt ein weiteres Dip-coating in einer Lösung bestehend aus 0,025 mol/l Ti(iso-C₃H₇O)₄ , 0,025 mol/l Si(OC₂H₅)₄ ünd 0,05 mol/l HCl in Form von 37%iger Salzsäure in iso-Propanol und 0,05 mol/l CaCl₂x2H₂O in Ethanol. . Die elektrochemische Abscheidung von Hydroxyapatit erfolgt nach ca. 4 Stunden unter den Bedingungen des Beispiels 1, jedoch bei 180 Hz.

### Beispiel 5

Eine Ti-Platte wie in Beispiel 1 wird einer zweimaligen Sol-Gel-Behandlung unter Verwendung einer Lösung bestehend aus 0,033 mol/l Ti(iso-C₃H₇O)₄ , 0,033 mol/l Si(OC₂H₅)₄ , 0,033 mol/l Al(iso-C₄H₉O)₃ ünd 0,1 mol/l HCl in Form von 37%iger Salzsäure in iso-Propanol nach der Dip-coating-Technik unterzogen (Ziehgeschwindigkeit 1 cm/min). Zusätzlich erfolgt ein weiteres Dip-coating unter Verwendung einer Lösung bestehend aus 0,0167 mol/l Ti(iso-C₃H₇O)₄ , 0,0167 mol/l Si(OC₂H₅)₄ , 0,0167 mol/l Al(iso-C₄H₉O)₃ ünd 0,1 mol/l HCl in Form von 37%iger Salzsäure in iso-Propanol und 0,05 mol/l CaCl₂x2H₂O in Ethanol. . Die elektrochemische Abscheidung von Hydroxyapatit erfolgt nach ca. 4 Stunden unter den Bedingungen des Beispiels 1, jedoch bei 50 Hz und 3,8 V. Während 7 Minuten wird eine Belegungsdichte von 1,98 mg/cm² erzielt.

### Beispiel 6

Eine mit 2 µm Ti beschichtete Glasplatte der Abmessung 80x20x1 mm wird in Analogie zu Beispiel 5 vorbehandelt und mit Hydroxyapatit beschichtet.

Die REM-Aufnahme (5 kV) eines Bruchkantenpräparates zeigt eine durchschnittliche Höhe der Beschichtung von 15 µm.

### Beispiel 7

Eine mit 2,5 µm TiN beschichtete Glasplatte wird gemäß Beispiel 6 behandelt.

Die REM-Aufnahme (5 kV) eines Bruchkantenpräparates zeigt eine durchschnittliche Höhe der Beschichtung von 10 µm.

### Beispiel 8

Eine mit 2 µm TiN beschichtete Edelstahlplatte mit den Abmessungen 80x20x1 mm wird einer Sol-Gel-Behandlung unter Verwendung einer Lösung bestehend aus 0,033 mol/l Ti(iso-C₃H₇O)₄ , 0,033 mol/l Si(OC₂H₅)₄ , 0,033 mol/l Al(iso-C₄H₉O)₃ ünd 0,1 mol/l HCl in Form von 37%iger Salzsäure in iso-Propanol nach der Dip-coating-Technik unterzogen (Ziehgeschwindigkeit 1 cm/min). Zusätzlich erfolgt ein weiteres Dip-coating unter Verwendung einer Lösung bestehend aus 0,0167 mol/l Ti(iso-C₃H₇O)₄ , 0,0167 mol/l Si(OC₂H₅)₄ , 0,0167 mol/l Al(iso-C₄H₉O)₃ ünd 0,1 mol/l HCl in Form von 37%iger Salzsäure in iso-Propanol und 0,05 mol/l CaCl₂x2H₂O in Ethanol. . Die elektrochemische Abscheidung von Hydroxyapatit erfolgt nach ca. 4 Stunden unter den Bedingungen des Beispiels 1, jedoch bei 50 Hz und 3,5 V. Während 5 Minuten wird eine Belegungsdichte von 1,46 mg/cm² erzielt.

### Beispiel 9

Eine mit 2 µm TiN beschichtete Edelstahlplatte mit den Abmessungen 80x20x1 mm wird einer Sol-Gel-Behandlung unter Verwendung einer Lösung bestehend aus 0,05 mol/l Ti(iso-C₃H₇O)₄, 0,05 mol/l Si(OC₂H₅)₄ ünd 0,1 mol/l HCl in Form von 37%iger Salzsäure in iso-Propanol nach der Dip-coating-Technik unterzogen (Ziehgeschwindigkeit 1 cm/min). Zusätzlich erfolgt ein weiteres Dip-coating in einer Lösung bestehend aus 0,025 mol/l Ti(iso-C₃H₇O)₄ , 0,025 mol/l Si(OC₂H₅)₄ ünd 0,05 mol/l HCl in Form von 37%iger Salzsäure in iso-Propanol und 0,05 mol/l CaCl₂x2H₂O in Ethanol. Die elektrochemische Abscheidung von Hydroxyapatit erfolgt nach ca. 4 Stunden unter den Bedingungen des Beispiels 1, jedoch bei 50 Hz, 4 V und 35 °C.

### Beispiel 10

Eine mit 2,2 µm TiN beschichtete Edelstahlplatte wird gemäß Beispiel 8 vorbehandelt . Die elektrochemische Abscheidung von Hydroxyapatit erfolgt nach ca. 4 Stunden unter den Bedingungen des Beispiels 1, jedoch bei 50 Hz, 3.8 V und 25 °C. Während 8 Minuten wird eine Belegungsdichte von 1,58.mg/cm² erzielt..

Eine mechanisch abgetragene Beschichtungsprobe wird 2 Stunden bei 200 °C getrocknet , in konzentrierter Salzsäure gelöst und spektrophotometrisch analysiert (Ca²⁺ : Arsenazo-III-Methode, PO₄³⁻ : Molybdänblau- Methode). Das Ca/P-Verhältnis beträgt 1,58.

### Beispiel 11

Eine mit 1,8 µm TiN beschichtete Poly(tetrafluorethen)-Platte mit den Abmessungen 70x20x5 mm wird gemäß Beispiel 9 vorbehandelt. Die elektrochemische Abscheidung von Hydroxyapatit erfolgt nach ca. 4 Stunden unter den Bedingungen des Beispiels 1, jedoch bei 50 Hz, 4 V und 25 °C. Während 7 Minuten wird eine Belegungsdichte von 1,28.mg/cm² erzielt.

### Beispiel 12

Eine mit 2,4 µm Titannioboxynitrid beschichtete Edelstahlplatte wird gemäß Beispiel 9 vorbehandelt. Die elektrochemische Abscheidung von Hydroxyapatit erfolgt nach ca. 4 Stunden unter den Bedingungen des Beispiels 1, jedoch bei 50 Hz, 3.5 V und 25 °C. Während 5 Minuten wird eine Belegungsdichte von 1,32.mg/cm² erzielt..

### Beispiel 13

Eine mit 2 µm Titanzirkoniumnitrid beschichtete Edelstahlplatte mit den Abmessungen 80x20x1 mm wird einer Sol-Gel-Beschichtung unter Verwendung einer Lösung bestehend aus 0,05 mol/l Ti(OC₄H₉)₄, 0,05 mol/l Zr(OC₄H₉)₄ ünd 0,1 mol/l HCl in Form von 37%iger Salzsäure in n-Butanol nach der Dip-coating-Technik unterzogen (Ziehgeschwindigkeit 1 cm/min). Zusätzlich erfolgt ein weiteres Dip-coating in einer Lösung bestehend aus 0,025 mol/l Ti(OC₄H₉)₄ , 0,025 mol/l Zr(OC₄H₉)₄ ünd 0,05 mol/l HCl in Form von 37%iger Salzsäure in n-Butanol und 0,05 mol/l CaCl₂x2H₂O in Ethanol. . Die elektrochemische Abscheidung von Hydroxyapatit erfolgt nach ca. 4 Stunden unter den Bedingungen des Beispiels 1, jedoch bei 50 Hz, 4 V und 25 °C.

### Beispiel 14

Eine TiAl6V4-Scheibe (Durchmesser 14 mm, Dicke 1 mm) wird einer Sol-Gel-Beschichtung mittels Ultraschallvernebelung ( 800 kHz, 200 W, Trägergas: N₂) unter Verwendung der in Beispiel 8 genannten Lösungen unterzogen. Die elektrochemische Abscheidung von Hydroxyapatit erfolgt nach ca. 6 Stunden unter den Bedingungen des Beispiels 1, jedoch bei 50 Hz, 4 V, 25 °C und mit einem Platinblech als Anode bzw. einem Platindraht als Katodenhalterung.

### Beispiel 15

Eine mit 2 µm TiN beschichtete TiAl6V4-Scheibe (Durchmesser 14 mm, Dicke 1 mm) wird einer Sol-Gel-Beschichtung mittels Ultraschallvernebelung ( 800 kHz, 200 W, Trägergas: N₂) unter Verwendung der in Beispiel 9 genannten Lösungen unterzogen. Die elektrochemische Abscheidung von Hydroxyapatit erfolgt nach ca. 6 Stunden unter den Bedingungen des Beispiels 1, jedoch bei 50 Hz, 3,5 V, 25 °C und mit einer Edelstahlklammer als Katodenhalterung.

### Beispiel 16

Eine Edelstahlplatte (1.4301) mit den Abmessungen 80x20x1 mm wird gemäß Beispiel 9 vorbehandelt. Die elektrochemische Abscheidung von Hydroxyapatit erfolgt nach ca. 4 Stunden unter den Bedingungen des Beispiels 1, jedoch bei 50 Hz, 4 V und 25 °C. Während 3 Minuten wird eine Belegungsdichte von 0,88.mg/cm² erzielt..

### Beispiel 17

Die gemäß den Beispielen 1 - 16 hergestellten Beschichtungspräparate werden eine Stunde bei 380 °C unter atmosphärischen Bedingungen getempert. Dabei tritt ein Masseverlust in der Größenordnung von 10% ein. Die so behandelten Beschichtungen sind carbonathaltig, ersichtlich aus den IR-Spektren von jeweils mechanisch entferntem Beschichtungsmaterial (ν_{CO} bei 1450 cm⁻¹)

## Patentansprüche

1. Verfahren zur Beschichtung von Metall-Legierungs- und Keramikoberflächen mit Hydroxylapatit, gekennzeichnet durch folgende Verfahrensschritte:
Vorbehandlung der Oberfläche durch Aufbringen einer Sol-Gel-Beschichtung unter Verwendung von Alkoxiden eines Elements oder mehrerer Elemente der 3. - 5. Haupt- oder Nebengruppe des Periodensystems und
elektrochemische Abscheidung von Hydroxylapatit der allgemeinen Zusammensetzung Caₓ₊₉(PO₄)ₓ₊₅(HPO₄)₁₋ₓ(OH)ₓ₊₁, wobei x für Werte zwischen Null und 1 steht, unter Verwendung eines calcium- und phosphathaltigen Elektrolyten mittels gepulstem Gleichstrom.

2. Verfahren nach Anspruch 1, dadurch gekennzeichnet,
daß als Metalle Titan, Gold, Platin und Legierungen, insbesondere Edelstähle, CoCr28Mo und TiAl6V4 verwendet werden.

3. Verfahren nach Anspruch 1, dadurch gekennzeichnet,
daß als keramische Werkstoffe Oxide, Nitride, Oxynitride bzw. Carbonitride, die in ihrer Zusammensetzung ein oder mehrere Metalle der 4. und 5. Nebengruppe des Periodensystems enthalten, verwendet werden.

4. Verfahren gemäß den Ansprüchen 1 bis 3, dadurch gekennzeichnet,
daß bei der Sol-Gel-Beschichtung zusätzlich Calcium- und/oder Phosphationen verwendet werden.

5. Verfahren gemäß den Ansprüchen 1 bis 4, dadurch gekennzeichnet,
daß die Sol-Gel-Beschichtung durch Sprühen, Ultraschallvernebelung oder nach der Dip-Coating- Methode aufgebracht wird.

6. Verfahren gemäß den Ansprüchen 1 bis 5, dadurch gekennzeichnet,
daß die elektrochemische Abscheidung unter Anwendung von gepulstem Gleichstrom mit einer Pulsfrequenz zwischen 1 und 300 Hz und einer Spannung zwischen 2 und 10 V durchgeführt wird.

7. Verfahren nach Anspruch 6, dadurch gekennzeichnet,
daß eine Pulsfrequenz von 50 Hz bei gleichen Ein-Aus-Intervallen angewendet wird.

8. Verfahren gemäß den Ansprüchen 1 bis 6, dadurch gekennzeichnet,
daß die elektrochemische Abscheidung bei Temperaturen zwischen 20 und 40°C erfolgt.

9. Verfahren gemäß den Ansprüchen 1 bis 8, dadurch gekennzeichnet,
daß die Oberflächen vor der Sol-Gel- Beschichtung passiviert werden.

10. Verfahren nach Anspruch 9 dadurch gekennzeichnet,
daß die Oberflächen mittels verdünnter Phosphorsäure passiviert werden.

11. Verfahren nach Anspruch 1, dadurch gekennzeichnet,
daß die beschichteten Substrate einer thermischen Nachbehandlung bei Temperaturen zwischen 300 und 500°C unterzogen werden.

## Claims

1. Process for coating metal, alloy and ceramic surfaces with hydroxyl apatite, characterised by the following stages of the process:
pretreatment of the surface by application of a sol-gel coating using alkoxides of an element or several elements from the third to fifth main or subsidiary group of the periodic system and
electrochemical deposition of hydroxyl apatite having the general composition
Caₓ₊₉(PO₄)ₓ₊₅(HPO₄)₁₋ₓ(OH)ₓ₊₁, wherein x represents values between zero and 1, using a calcium- and phosphate-containing electrolyte by means of a pulsed direct current.

2. Process according to claim 1, characterised in that titanium, gold, platinum and alloys, in particular special steels, CoCr28Mo and TiAl6V4 are used as metals.

3. Process according to claim 1, characterised in that oxides, nitrides, oxynitrides and carbonitrides which contain one or more metals from the fourth and fifth subsidiary group of the periodic system in their composition are used as ceramic materials.

4. Process according to claims 1 to 3, characterised in that calcium and/or phosphate ions are additionally used in the sol-gel coating.

5. Process according to claims 1 to 4, characterised in that the sol-gel coating is applied by spraying, ultrasonic atomisation or by the dip-coating method.

6. Process according to claims 1 to 5, characterised in that electrochemical deposition is carried out using pulsed direct current with a pulse frequency between 1 and 300 Hz and a voltage between 2 and 10 V.

7. Process according to claim 6, characterised in that a pulse frequency of 50 Hz with equal on/off intervals is applied.

8. Process according to claims 1 to 6, characterised in that electrochemical deposition takes place at temperatures between 20 and 40°C.

9. Process according to claims 1 to 8, characterised in that the surfaces are passivated prior to sol-gel coating.

10. Process according to claim 9, characterised in that the surfaces are passivated using dilute phosphoric acid.

11. Process according to claim 1, characterised in that the coated substrates are subjected to a thermal after-treatment at temperatures between 300 and 500°C.

## Revendications

1. Procédé pour le revêtement de surfaces métalliques et de céramiques avec de l'hydroxylapatite, caractérisé par les étapes suivantes :
prétraitement de la surface par application d'un revêtement sol-gel en utilisant des alcoxydes d'un élément ou de plusieurs éléments du 3ème - 5ème Groupe principal ou Groupe secondaire de la Classification Périodique des Eléments et
séparation électrochimique de l'hydroxylapatite de la composition générale Caₓ₌₉(PO₄)ₓ₊₅(HPO₄)₁₋ₓ(OH)ₓ₊₁, x se situant pour des valeurs entre 0 et 1, en utilisant des électrolytes contenant du calcium et du phosphate au moyen d'un courant continu pulsé.

2. Procédé selon la revendication 1, caractérisé en ce qu'en tant que métaux, on utilise du titane, de l'or, du platine, des alliages, en particulier des aciers nobles, CoCr28Mo et TiAl6V4.

3. Procédé selon la revendication 1, caractérisé en ce qu'en tant que matériau céramique, on utilise des oxydes, nitrides, oxynitrides ou carbonitrides qui contiennent dans leurs compositions un ou plusieurs métaux du 4ème et 5ème Groupes secondaires de la Classification Périodique des Eléments.

4. Procédé selon les revendications 1 à 3, caractérisé en ce que pour le revêtement sol-gel, on utilise de plus des ions calcium et/ou phosphate.

5. Procédé selon les revendications 1 à 4, caractérisé en ce que le revêtement sol-gel est appliqué par pulvérisation, nébulisation aux ultrasons ou selon les procédés de Dip-Coating (revêtement au trempé).

6. Procédé selon les revendications 1 à 5, caractérisé en ce que la séparation électrochimique est effectuée en utilisant du courant continu pulsé avec une fréquence de pulsations entre 1 et 300 Hz et une tension entre 2 et 10 V.

7. Procédé selon la revendication 6, caractérisé en ce que l'on utilise une fréquence de pulsations de 50 Hz pour des intervalles identiques de "marche" - "arrêt".

8. Procédé selon les revendications 1 à 6, caractérisé en ce que la séparation électrochimique s'effectue à des températures entre 20 et 40°C.

9. Procédé selon les revendications 1 à 8, caractérisé en ce que les surfaces du revêtement sol-gel sont passivées.

10. Procédé selon la revendication 9, caractérisé en ce que les surfaces sont passivées au moyen d'acide phosphorique dilué.

11. Procédé selon la revendication 1, caractérisé en ce que les substrats revêtus sont soumis à un traitement thermique ultérieur à des températures entre 300 et 500°C.
